# EUROPÄISCHE PATENTANMELDUNG

(11) **EP 0 839 575 A2**
(43) Veröffentlichungstag der Anmeldung: **06.05.1998**
(21) Anmeldenummer: 97118717.4
(22) Anmeldetag: 28.10.1997
(51) Int. Cl.: B01J 23/89

(54) **Katalysatoren für die Aminierung von Alkylenoxiden, Alkoholen, Aldehyden und Ketonen**

(30) Priorität: 31.10.1996 DE 19645047
(71) Anmelder: BASF AKTIENGESELLSCHAFT, 67056 Ludwigshafen (DE)
(72) Erfinder: Wulff-Döring, Joachim, Dr., 67227 Frankenthal (DE); Melder, Johann-Peter, Dr., 67141 Neuhofen (DE); Schulz, Gerhard, Dr., 67069 Ludwigshafen (DE); Voit, Guido, Dr., 69198 Schriesheim (DE); Gutschoven, Frank, 3600 Gent (BE); Harder, Wolfgang, Dr., 69469 Weinheim (DE)
(74) Vertreter: Isenbruck, Günter, Dr.

(57) **Zusammenfassung**

Der Katalysator umfaßt, bezogen auf das Gesamtgewicht des Katalysators
- mehr als 6 - 50 Gew.-%: Kobalt, Nickel oder deren Gemisch,
- 0,001 - 25 Gew.-%: Ruthenium,
- 0 - 10 Gew.-%: Kupfer
und
- 0 - 5 Gew.-%: Promotoren
auf einem porösen Metalloxidträger.

Er ist herstellbar durch
(a) Imprägnieren des Trägers mit den Metallen, Promotoren oder Verbindungen davon,
(b) Trocknen und Calcinieren des imprägnierten Trägers und
(c) Reduzieren des calcinierten Trägers im Wasserstoffstrom,
wobei der Träger nicht mit Halogenverbindungen imprägniert wird.

## Beschreibung

Die Erfindung betrifft Ruthenium, Nickel und/oder Kobalt enthaltende Katalysatoren, die zur Aminierung von Alkylenoxiden, Alkoholen, Aldehyden und Ketonen verwendet werden können. Weiterhin betrifft die Erfindung Verfahren zur Herstellung der Katalysatoren, deren Verwendung in Aminierungsreaktionen und Verfahren zur Herstellung von Aminierungsprodukten.

Aus der EP-A-0 146 508 sind Dehydrierungs-/Hydrierungskatalysatoren bekannt, die Ruthenium, Nickel und/oder Kobalt enthalten. Die verwendeten Katalysatoren enthalten ca. 0,25 - 1,0 Gew.-% Ruthenium und 7,5 oder 10 Gew.-% Nickel oder 10 Gew.-% Kobalt oder 4 Gew.-% Nickel und 4 Gew.-% Kobalt, bezogen auf das Gesamtgewicht des Katalysators, auf einem Aluminiumoxidträger. Die Katalysatoren können neben Nickel auch Kupfer und Chrom, sowie neben Nickel und Kobalt auch Eisen enthalten. Das Ruthenium wird auf den Katalysator in Form einer Losung eines Rutheniumhalogenids aufgebracht. Der Katalysator wird beispielsweise zur Aminierung von Monoethanolamin mit Ammoniak verwendet, wobei die Umsetzung in einem Autoklaven durchgeführt wird.

Aus der EP-A-0 254 335 ist ein Verfahren zur Herstellung eines Hydrierungs- und/oder Dehydrierungskatalysators bekannt, wobei ca. 10 Gew.-% Nickel oder Kobalt und ca. 0,5 Gew.-% Ruthenium, bezogen auf das Gesamtgewicht des Katalysators, auf einen Aluminiumoxidträger aufgebracht werden. Der Katalysator wird durch Imprägnieren des Trägers mit einer Nickel- oder Kobaltnitratlösung, anschließender Tränkung mit wäßriger Salzsäure und anschließender Tränkung mit einer Losung von Rutheniumnitrosylnitrat hergestellt. Zur Imprägnierung des Trägers wird kein Rutheniumhalogenid verwendet. Der Katalysator wird zur Umsetzung von Monoethanolamin mit Ammoniak in einem Autoklaven eingesetzt.

Die bekannten Katalysatoren weisen durch die Tränkung eingebrachte Gehalte an Chloriden auf. Auf dem Katalysatorträger vorliegende Chloride können zudem sowohl bei der Katalysatorherstellung als auch bei Umsetzungen mit dem Katalysator zu Korrosionsproblemen führen, wenn beispielsweise Salzsäure aus dem Katalysator freigesetzt wird. Zudem ist insbesondere bei den in EP-A-0 254 335 beschriebenen Katalysatoren die Haltbarkeit unzureichend. Das Tränken des Trägers mit einer wäßrigen Salzsäurelösung, wie es in EP-A-0 254 335 beschrieben ist, führt zu verschlechterten mechanischen Eigenschaften des Katalysators, da die oxidischen Träger von der Säure angegriffen werden. Hierdurch wird die mechanische Stabiltät des Katalysators so vermindert, daß die Standzeit des Katalysators im Betrieb aufgrund von Katalysatorzerfall stark sinkt. Die bei der Umsetzung von Monoethanolamin mit Ammoniak erhaltenen Selektivitäten in bezug auf Ethylendiamin sind bei kontinuierlicher Verfahrensführung unzureichend.

Aufgabe der vorliegenden Erfindung ist die Bereitstellung von Aminierungskatalysatoren, die die Nachteile der bekannten Katalysatoren vermeiden und insbesondere keine Korrosion verursachen bei gleichzeitig guter Haltbarkeit und hoher Selektivität in bezug auf Ethylendiamin bei der Aminierung von Monoethanolamin mit Ammoniak.

Die Aufgabe wird durch Bereitstellung eines Katalysators gelöst, umfassend bezogen auf das Gesamtgewicht des Katalysators
- mehr als 6 - 50 Gew.-%: Kobalt, Nickel oder deren Gemisch,
- 0,01 - 25 Gew.-%: Ruthenium,
- 0 - 10 Gew.-%: Kupfer
und
- 0 - 5 Gew.-%: Promotoren aus der Gruppe Eisen, Rhodium, Palladium, Platin, Iridium, Osmium, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder Gemischen davon
auf einem porösen Metalloxidträger, der herstellbar ist durch
(a) Imprägnieren des Trägers mit den Metallen, Promotoren oder Verbindungen davon,
(b) Trocknen und Calcinieren des imprägnierten Trägers und
(c) Reduzieren des calcinierten Trägers im Wasserstoffstrom,
   wobei der Träger nicht mit Halogenverbindungen imprägniert wird.

Als Träger des Katalysators wird ein poröses Metalloxid eingesetzt, das vorzugsweise ausgewählt ist aus Aluminiumoxid, Alumosilicaten, Titandioxid, Zirkoniumdioxid, Magnesiumoxid oder Gemischen davon. Vorzugsweise werden Träger verwendet, die Aluminiumoxid enthalten und besonders bevorzugt aus Aluminiumoxid bestehen.

Auf den Katalysatorträger sind mehr als 6 - 50, vorzugsweise mehr als 6 bis 40, insbesondere mehr als 6 - 20 Gew.-% Kobalt, Nickel oder deren Gemisch aufgebracht. Die Gewichtsangaben beziehen sich dabei auf das Gesamtgewicht des Katalysators, sofern nichts anderes angegeben ist. Vorzugsweise sind auf den Träger mehr als 3 - 25, besonders bevorzugt mehr als 3 - 20, insbesondere mehr als 3 - 10, speziell 5 - 10 Gew.-% Kobalt aufgebracht. Ebenso sind vorzugsweise mehr als 3 - 25, besonders bevorzugt mehr als 3 - 20, insbesondere mehr als 3 - 10, speziell 5 - 10 Gew.-% Nickel aufgebracht.

Der Katalysator enthält außerdem 0,001 - 25, oft 0,01 - 25, vorzugsweise 0,1 - 10, besonders bevorzugt 0,5 - 5, insbesondere 0,5 - 2 Gew.-% Ruthenium auf dem Träger.

Zudem kann Kupfer auf den Träger aufgebracht sein. Der Gehalt an Kupfer beträgt dabei 0 - 10, vorzugsweise 0,1 - 10, besonders bevorzugt 0,5 - 5 Gew.-%.

Zudem können auf dem Träger 0 - 5 Gew.-% Promotoren vorliegen. Sie sind ausgewählt aus der Gruppe Eisen, Rhodium, Palladium, Platin, Iridium, Osmium, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder Gemischen davon.

Besonders bevorzugt enthält der Katalysator 5 - 10 Gew.-% Kobalt, 5 bis 10 Gew.-% Nickel, 0,5 - 5 Gew.-% Ruthenium und 0,5 - 5 Gew.-% Kupfer. Insbesondere beträgt der Gehalt an Nickel 7 - 9 Gew.-%, Kobalt 7 bis 9 Gew.-%, Kupfer 1,3 - 1,9 Gew.-% und Ruthenium 0,5 - 1,5 Gew.-%.

Durch zusätzliche Dotierung der Katalysatorträger mit den vorstehend aufgeführten Promotoren kann die Selektivität des Katalysators gesteuert werden. Vorzugsweise beträgt der Gehalt an Promotor 0,001 - 5, insbesondere 0,01 - 3 Gew.-%.

Die Katalysatoren werden hergestellt durch
(a) Imprägnierung des Trägers mit den Metallen, Promotoren oder Verbindungen davon,
(b) Trocknen und Calcinieren des imprägnierten Trägers und
(c) Reduzieren des calcinierten Trägers im Wasserstoffstrom.

Im folgenden werden die Metalle und die Promotoren abgehandelt und gemeinsam als Metalle bezeichnet.

Die Imprägnierung des Trägers mit den Metallen oder Verbindungen der Metalle im Schritt (a) kann durch beliebige geeignete Verfahren erfolgen. Beispielsweise kann der Träger mit einer Losung der Metallverbindungen getränkt werden. Er kann auch durch Aufsprühen der Losungen oder durch gemeinsames Verkneten oder durch Ausfällung der Metalle oder Verbindungen auf den Träger imprägniert werden.

Die Metalle, mit denen der Träger imprägniert wird, werden dabei vorzugsweise in Form einer Losung aus den halogenidfreien Salzen der Metalle eingesetzt. Beispielsweise können die Nitrate, Formiate, Oxalate oder Ammoniakate, vorzugsweise die Oxalate und Nitrate, besonders bevorzugt die Nitrate eingesetzt werden.

Der Träger wird nicht mit Halogenverbindungen imprägniert. Dabei wird der Träger insbesondere nicht mit Metallhalogeniden oder Losungen der Metallhalogenide oder mit einer anderen halogenidhaltigen Lösung, wie wäßriger Salzsäurelösung, imprägniert.

Hierdurch werden die eingangs beschriebenen Probleme vermieden, und es wird ein vorteilhafter Katalysator erhalten.

Die Imprägnierung des Katalysators mit den Metallen oder Metallsalzlösungen kann in beliebiger Reihenfolge erfolgen. Beispielsweise kann der Katalysatorträger mit einer Lösung, die sämtliche Metallsalze enthält, imprägniert werden. Der Träger kann auch mit mehreren Lösungen, die Salze eines oder mehrerer der verwendeten Metalle enthalten, nacheinander imprägniert werden. Dabei können alle oder einzelne Imprägnierungsschichte mehrfach ausgeführt werden, wobei die Reihenfolge der Imprägnierungen verändert werden kann. Beim mehrfachen Imprägnieren, insbesondere beim mehrfachen Aufsprühen der Lösungen oder bei mebrfacher Tränkung des Trägers mit den Lösungen kann die Konzentration der Metallsalze in den Lösungen gering gehalten werden.

Die Konzentration in den Lösungen bzw. der Lösung kann auch so eingestellt werden, daß durch einfaches Aufbringen oder Tränken die gewünschte Metallmenge auf dem Träger vorliegt. Nach dem Imprägnieren des Trägers mit den Metallen oder Verbindungen der Metalle wird der imprägnierte Träger vorzugsweise bei Temperaturen von 80 - 150°C, besonders bevorzugt von 80 - 120°C getrocknet. Sodann wird der imprägnierte Träger bei Temperaturen von 150 - 500°C, vorzugsweise 300 - 500°C calciniert. Anschließend wird der imprägnierte und calcinierte Träger im Wasserstoffstrom bei einer Temperatur von 150 - 500°C, vorzugsweise 200 - 400°C reduziert. Dazu wird der Träger nach dem Calcinieren vorzugsweise zunächst abgekühlt. Der Wasserstoffstrom kann als reiner Wasserstoffstrom oder als verdünnter Wasserstoffstrom, beispielsweise in einem Inertgas, wie Stickstoff eingesetzt werden.

Die Reduktion, die auch als Aktivierung bezeichnet werden kann, kann direkt im Reaktor durchgeführt werden, der auch für die nachfolgende Synthese eingesetzt wird. Wird die Reduktion der Katalysatoren in einem separaten Reaktor durchgeführt, so werden die Katalysatoren vor dem Ausbau aus dem Reaktor vorzugsweise bei Temperaturen von 10 - 60, insbesondere 20 - 40°C mit einem Gasgemisch, das freien Sauerstoff enthält, oberflächlich passiviert. Die so passivierten Katalysatoren können nach dem Einbau in den zur Synthese vorgesehenen Reaktor entweder in einem Wasserstoffstrom, vorzugsweise Stickstoff-/Wasserstoffstrom bei einer Temperatur von 150 - 200°C, vorzugsweise 170 - 190°C aktiviert werden. Sie können auch ohne weitere Aktivierung eingesetzt werden.

Die erfindungsgemäßen Katalysatoren können in jeder geeigneten Form eingesetzt werden, beispielsweise als Formkörper, wie Stränge oder Pellets, oder in Pulverform. Dabei können vorgeformte Träger mit den Metallen oder Metallverbindungen imprägniert werden, oder imprägnierte Träger in die gewünschte Form gebracht werden. Bei dem gemeinsamen Verkneten oder der gemeinsamen Fällung von Trägermaterial und Metallen oder Metallverbindungen werden die resultierenden Massen in der Regel anschließend verformt. Insbesondere bei kontinuierlicher Verfahrensführung werden die Katalysatoren in Form von Formkörpern verwendet.

Die erfindungsgemäßen Katalysatoren können in einer Vielzahl von Umsetzungen eingesetzt werden. Vorzugsweise werden sie erfindungsgemäß in Hydrierungsreaktionen, Dehydrierungsreaktionen oder Hydrierungs-/Dehydrierungsreaktionen eingesetzt. Insbesondere werden die Katalysatoren zur Aminierung von Alkylenoxiden, Alkoholen, Aldehyden oder Ketonen mit Ammoniak, primären oder sekundären Aminen eingesetzt. Besonders bevorzugt werden die Katalysatoren zur Aminierung von Alkoholen, speziell bei der Umsetzung von Monoethanolamin mit Ammoniak zu Ethylendiamin eingesetzt. Dabei sind die erfindungsgemäßen Katalysatoren über eine lange Zeit mechanisch stabil und zeigen keinen Abfall der Aktivität. Sie zeigen bei gleicher Aktivität wie die bekannten Katalysatoren mit hohem Gehalt an Nickel und Kobalt eine höhere Selektivität in bezug auf die Bildung primärer Aminierungsprodukte bei der Aminierung mit Ammoniak. Sie weisen zudem eine wesentlich verbesserte Standzeit auf und geben keine korrosiven Verbindungen ab.

Die Erfindung betrifft auch ein Verfahren zur Herstellung von Aminierungsprodukten durch Umsetzung von Alkylenoxiden, Alkoholen, Aldehyden oder Ketonen mit Ammoniak, primären oder sekundären Aminen in Gegenwart von freiem Wasserstoff und in Gegenwart eines wie vorstehend beschriebenen Katalysators.

Die Aminierung erfolgt dabei durch Umsetzung von Ammoniak oder einer primären oder sekundären Aminogruppe mit einer Hydroxylgruppe, Aldehyd- oder Ketogruppe oder einer Alkylenoxidgruppe, wobei Aldehyd-, Keto- oder Alkylenoxidgruppen reduktiv aminiert werden beziehungsweise Hydroxylgruppen durch Aminogruppen ersetzt werden. Dabei können die Aminogruppen und zu aminierenden Gruppen in unterschiedlichen Molekülen oder auch im gleichen Molekül vorliegen. Liegen die miteinander umzusetzenden Gruppen im gleichen Molekül vor, so können cyclische Verbindungen resultieren. Die Verbindungen, in denen Aminogruppen und zu aminierende Gruppen vorliegen, können zudem entweder als Aminkomponente oder als zu aminierende Verbindung wirken.

Beispielsweise können bei der Aminierung von Monoethanolamin (MEA) mit Ammoniak unter anderen Ethylendiamin (EDA), Aminoethylethanolamin (AEEA), Diethylentriamin (DETA) und Piperazin erhalten werden.

Es können auch Verbindungen eingesetzt werden, die zwei oder mehrere der Hydroxylgruppen, Aldehyd-, Keto- oder Alkylenoxidgruppen aufweisen. Dabei können auch gemischte Gruppen vorliegen. Insbesondere können Diole oder Polyole, insbesondere Ethylenglykole eingesetzt werden. Weiterhin können auch Verbindungen eingesetzt werden, die mehrere primäre oder sekundäre Aminogruppen aufweisen, wie Alkylendiamine, insbesondere Ethylendiamin. Beispielsweise können Ethylenglykole oder Ethanolamine mit oder in Gegenwart von Ammoniak, Ethanolaminen, Ethylendiaminen oder Diethylentriaminen aminiert werden.

Gemäß einer Ausführungsform der Erfindung dient das erfindungsgemäße Verfahren zur Herstellung von Aminen der allgemeinen Formel (I)

R¹R²N-CHR³R⁴ (I)

in der R¹, R², R³ und R⁴ unabhängig Wasserstoff, C₁₋₂₀₀-Alkyl, C₃₋₁₂-Cycloalkyl, durch Amino, C₁₋₂₀-Alkylamino, (Di-C₁₋₂₀-alkyl)amino und/oder Hydroxy substituiertes C₁₋₂₀-Alkyl, C₂₋₃₀-Alkoxyalkyl, R⁵(OCHR⁶CH₂)ₙ, Aryl, C₇₋₂₀-Aralkyl oder C₇₋₂₀-Alkylaryl bedeuten oder R¹ oder R² gemeinsam (CH₂)ₗ-X-(CH₂)ₘ bilden,
- wobei: R⁵ Wasserstoff oder C₁₋₄-Alkyl,
R⁶ Wasserstoff oder Methyl,
X Sauerstoff oder NR⁶ bedeuten,
n eine ganze Zahl von 2 - 30,
l eine ganze Zahl von 2 - 4,
m eine ganze Zahl von 1 - 4 ist,
aus primären oder sekundären Alkoholen, Ketonen, Aldehyden der allgemeinenen Formel (II)

R³R⁴CHOH oder R³R⁴CO (II)

in der R³ und R⁴ die vorstehend angegebene Bedeutung haben, und primären oder sekundären Aminen der allgemeinen Formel (III)

R¹R²NH (III)

in der R¹ und R² die vorstehend angegebene Bedeutung haben.

Die Reste R¹, R², R³, R⁴, insbesondere die Reste R¹ und R² können dabei C₁₋₂₀₀-Alkyl, vorzugsweise C₁₋₈-Alkyl sein, wie Methyl, Ethyl, n-Propyl, Isopropyl, n-Butyl, Isobutyl, sec.-Butyl, tert.-Butyl, n-Pentyl, Isopentyl, sec.-Pentyl, neo-Pentyl, 1,2-Dimethylpropyl, n-Hexyl, Isohexyl, sec.-Hexyl, n-Heptyl, Isoheptyl, n-Octyl, Isooctyl, 2-Ethylhexyl, n-Decyl, 2-n-Propyl-n-heptyl, n-Tridecyl, 2-n-Butyl-n-nonyl, 3-n-Butyl-n-nonyl, besonders bevorzugt Isopropyl, 2-Ethylhexyl, n-Decyl, 2-n-Propyl-n-heptyl, n-Tridecyl, 2-n-Butyl-n-nonyl und 3-n-Butyl-n-nonyl, sowie C₄₀₋₂₀₀-Alkyl, wie Polybutyl, Polyisobutyl, Polypropyl, Polyisopropyl und Polyethyl, besonders bevorzugt Polybutyl und Polyisobutyl.

R¹ und R² können gemeinsam (CH₂)ₗ-X-(CH₂)ₘ sein, wobei X Sauerstoff oder N-R⁶ ist mit der nachstehend angegebenen Bedeutung und l eine ganze Zahl von 2 - 4, wie 2, 3 oder 4, bevorzugt 2 oder 3, besonders bevorzugt 2 und m eine ganze Zahl von 1 - 4, wie 1, 2, 3 oder 4, bevorzugt 2, 3 oder 4, besonders bevorzugt 2 oder 3 ist.

R¹, R², R³, R⁴ können C₃₋₁₂-Cycloalkyl, bevorzugt C₃₋₈-Cycloalkyl sein, wie Cyclopropyl, Cyclobutyl, Cyclopentyl, Cyclohexyl, Cycloheptyl, Cyclooctyl, besonders bevorzugt Cyclopentyl, Cyclohexyl und Cyclooctyl.

Sie können Aryl sein, wie Phenyl, 1-Naphthyl, 2-Naphthyl, 1-Anthryl, 2-Anthryl, 9-Antryl, bevorzugt Phenyl, 1-Naphthyl, 2-Naphthyl, besonders bevorzugt Phenyl.

Sie können C₇₋₂₀-Alkylaryl, bevorzugt C₇₋₁₂-Alkylphenyl sein, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl, 4-n-Propylphenyl.

Sie können C₇₋₂₀-Aralkyl, bevorzugt C₇₋₁₂-Phenylalkyl sein, wie Benzyl, 1-Phenylethyl, 2-Phenylethyl, 1-Phenylpropyl, 2-Phenylpropyl, 3-Phenylpropyl, 1-Phenylbutyl, 2-Phenylbutyl, 3-Phenylbutyl, 4-Phenylbutyl, besonders bevorzugt Benzyl, 1-Phenylethyl und 2-Phenylethyl.

R¹ kann insbesondere ein C₁₋₂₀-Alkyl, bevorzugt C₁₋₈-Alkyl sein, wie Methyl, Ethyl, n-Propyl, Isopropyl, n-Butyl, Isobutyl, 6-Butyl, tert.-Butyl, n-Pentyl, Isopentyl, sec.-Pentyl, neo-Pentyl, 1,2-Dimethylpropyl, n-Hexyl, Isohexyl, sec.-Hexyl, n-Heptyl, Isoheptyl, n-Octyl, Isooctyl, besonders bevorzugt C₁₋₄-Alkyl, wie Methyl, Ethyl, n-Propyl, Isopropyl, n-Butyl, Isobutyl, sec.-Butyl, tert.-Butyl.

R³ und R⁴ können insbesondere C₁₋₂₀-Hydroxyalkyl, bevorzugt C₁₋₈-Hydroxyalkyl sein, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl, 1-Hydroxymethylethyl, durch Amino und/oder Alkylamino und/oder Dialkylamino und/oder Hydroxy substituiertes C₁₋₂₀-Alkyl, bevorzugt C₁₋₈-Alkyl, besonders bevorzugt C₁₋₄-Alkyl, wie N-(Hydroxyethyl)aminoethyl und N-(Aminoethyl)aminoethyl.

Sie können C₂₋₃₀-, bevorzugt C₂₋₂₀-, besonders bevorzugt C₂₋₈-Alkoxyalkyl sein, wie Methoxymethyl, Ethoxymethyl, n-Propoxymethyl, Isopropoxymethyl, n-Butoxymethyl, Isobutoxymethyl, sec.-Butoxymethyl, tert.-Butoxymethyl, 1-Methoxymethyl, 2-Methoxymethyl, besonders bevorzugt C₂₋₄-Alkoxyalkyl, wie Methoxymethyl, Ethoxymethyl, n-Propoxymethyl, Isopropoxymethyl, n-Butoxymethyl, Isobutoxymethyl, sec.-Butoxymethyl, tert.-Butoxymethyl, 1-Methoxyethyl und 2-Methoxyethyl.

Sie können R⁵-(OCHR⁶CH₂)ₙ sein, wobei R⁵ C₁₋₄-Alkyl ist, wie Methyl, Ethyl, n-Propyl, Isopropyl, n-Butyl, Isobutyl, sec.-Butyl, tert.-Butyl, bevorzugt Methyl oder Ethyl, besonders bevorzugt Methyl. R⁶ ist dabei Wasserstoff oder Methyl.

n ist eine ganze Zahl von 2 - 10, bevorzugt von 2 - 8, wie 2, 3, 4, 5, 6, 7 oder 8, besonders bevorzugt 2, 3, 4, 5, 6.

Alle Reste R¹ - R⁶ können auch Wasserstoffatome sein.

Das erfindungsgemäße Verfahren kann diskontinuierlich oder bevorzugt kontinuierlich durchgeführt werden. Bei der kontinuierlichen Durchführung wird dabei vorzugsweise in einem Rohrreaktor in Riesel- oder Sumpffahrweise gearbeitet. Insbesondere bei der Aminierung von Alkoholen wird bei Temperaturen von 120 - 250°C, bevorzugt 150 - 190°C und Drücken von 150 - 300 bar, bevorzugt 180 - 220 bar gearbeitet. Allgemein kann bei Temperaturen von 80 - 250°C und Drücken von 100 - 400 bar gearbeitet werden.

Bei der Umsetzung mit Ammoniak kann Ammoniak als Reagenz und als Lösungsmittel eingesetzt werden. Dabei können pro Mol Alkylenoxid, Alkohol, Aldehyd oder Keton 1 - 20, vorzugsweise 6 - 12 Mol Ammoniak eingesetzt werden. Die Katalysatorbelastung beträgt vorzugsweise 0,05 bis 2,0, besonders bevorzugt 0,1 - 1,0 kg Alkylenoxid, Alkohol, Aldehyd oder Keton pro Liter Katalysator pro Stunde. Die Umsetzung kann auch in Gegenwart von Wasser durchgeführt werden, wobei bis zu 15 Gew.-%, bezogen auf das gesamte Reaktionsgemisch, Wasser zugesetzt werden können.

Die Verwendung der erfindungsgemäßen Katalysatoren im erfindungsgemäßen Verfahren erlaubt insbesondere bei der Umsetzung von Monoethanolamin mit Ammoniak eine hohe Ausbeute an Ethylendiamin, bedingt durch die hohe Selektivität der erfindungsgemäßen Katalysatoren.

Die nach dem erfindungsgemäßen Verfahren erhaltenen Produkte eignen sich u.a. als Zwischenprodukte bei der Herstellung von Kraftstoffadditiven, beispielsweise beschrieben in US 3,275,554, DE-A-21 25 039 oder DE-A-36 11 230. Ebenso können die erfindungsgemäß erhaltenen Verbindungen eingesetzt werden bei der Herstellung von Tensiden, Arznei- und Pflanzenschutzmitteln, sowie Vulkanisationsbeschleunigern. Nachstehend wird die Erfindung zusätzlich anhand von Beispielen erläutert.

### BEISPIEL 1

135 g Al₂O₃-Stränge mit einem Durchmesser von 4 mm (D10-10, hergestellt von der BASF AG, Ludwigshafen) wurden mit 88 ml einer wäßrigen Tränklösung, die 8,88 g NiO, 8,88 g CoO und 3,55 g CuO enthielt, unter mehrmaligem guten Durchmischen beim Raumtemperatur für zwei Stunden stehen gelassen. Der Katalysatorvorläufer wurde für 16 Stunden bei 120°C getrocknet und für vier Stunden bei 400°C calciniert. Dieser Vorgang wurde wiederholt. Sodann wurden 140 g dieser Stränge in eine Tränktrommel gegeben und 73,5 ml einer wäßrigen Rutheniumnitratlösung, die 1,57 g Ruthenium enthielt, innerhalb von 10 Minuten aufgesprüht. Nickel Kobalt und Kupfer wurden dabei in Form der Nitrate eingesetzt.

Anschließend wurde der Katalysatorvorläufer 16 Stunden bei 120°C getrocknet und für vier Stunden bei 400°C calciniert. Nach dem Abkühlen wurden die Stränge in eine Reduktionsapparatur eingebaut und für zwei Stunden mit 20 l N₂/h gespült. Danach wurde mit einer Rate von 2°C/min und 20 l H₂/h Wasserstofffluß auf 300°C aufgeheizt und 20 Stunden bei dieser Temperatur gehalten. Nach Abkühlen im Stickstoffstrom wurde der Katalysator mit einem Luft/Stickstoff-Gemisch passiviert, wobei die Temperaturerhöhung maximal 20°C betragen darf. Der so hergestellte Katalysator enthielt 1 Gew.-% Ruthenium, 7,9 Gew.-% Nickel, 7,9 Gew.-% Kobalt und 3,2 Gew.-% Kupfer auf Aluminiumoxid. Die Ergebnisse bei der Aminierung von Monoethanolamin sind in Tabelle 1 zusammengestellt. Der Katalysator war nach einer Betriebszeit von 303 Stunden noch vollständig erhalten.

### Vergleichsbeispiel V1

Der Katalysator wurde nach dem in EP-A-0 254 335, Beispiel 1 angegebenen Verfahren hergestellt. Der so erhaltene Katalysator enthielt 10 Gew.-% Nickel und 0,5 Gew.-% Ruthenium auf Aluminiumoxid (D10-10 der BASF AG, Ludwigshafen). Die Ergebnisse bei der Aminierung von Monoethanolamin sind in Tabelle 1 zusammengestellt. Der Katalysator war nach einer Betriebszeit von 48 Stunden vollständig zerfallen.

### Vergleichsbeispiel V2

Der Katalysator wurde nach dem in EP-A-0 254 335, Beispiel 13 angegebenen Verfahren hergestellt. Der so erhaltene Katalysator enthielt 10 Gew.-% Kobalt, 0,5 Gew.-% Ruthenium auf Aluminiumoxid (D10-10 der BASF AG, Ludwigshafen). Die Ergebnisse bei der Aminierung von Monoethanolamin sind in Tabelle 1 zusammengestellt. Der Katalysator war nach einer Betriebszeit von 48 Stunden vollständig zerfallen.

### Aminierung:

Die Aminierung von Monoethanolamin in Gegenwart der Katalysatoren des Beispiels 1 und der Vergleichsbeispiele V1 und V2 wurde wie folgt durchgeführt: ein 100 ml fassender Rohrreaktor mit einer Länge von 55 cm und einem Innendurchmesser von 1,5 cm wurde mit 50 g passiviertem Katalysator befüllt und der Katalysator bei 180°C zunächst mit einem Gemisch aus 20 Vol.-% Wasserstoff / 80 Vol.-% Stickstoff und anschließend mit 100 Vol.-% Wasserstoff aktiviert. Nach Einstellung der Reaktionstemperatur auf 175 - 195°C in Abhängigkeit von der Aktivität des Katalysators wurde der Reaktor mit 10 - 30 g/h Monoethanolamin, 20 - 70 g/h Ammoniak und 3 - 10 Nl/h Wasserstoff beschickt. Durch gaschromatographische Analyse des Austrags wurden der Umsatz und die Selektivität bezüglich der Komponenten Ethylendiamin (EDA), Aminoethylethanolamin (AEEA), Diethylentriamin (DETA) und Piperazin bestimmt. Die Katalysatorbelastung ist in bezug auf Monoethanolamin (MEA) angegeben. Die Ergebnisse sind in der nachstehenden Tabelle 1 zusammengefaßt.

### BEISPIEL 2

Es wurde der Katalysator verwendet, der in Beispiel 1 beschrieben ist.

### Aminierung:

Die Aminierung von Monoethanolamin in Gegenwart des Katalysators wurde wie folgt durchgeführt: ein 1 l fassender Rohrreaktor wurde mit 500 ml passiviertem Katalysator zwischen zwei Schichten mit je 250 ml V2A-Ringen befüllt und ohne weitere Vorbehandlung angefahren. Nach Einstellung der Reaktionstemperatur auf 155 - 200°C in Abhängigkeit von der Aktivität des Katalysators wurde der Reaktor mit 130 - 200 g/h MEA bei einem Verhältnis von MEA zu NH₃ von 1:8 und mit 10 - 50 Nl/h Wasserstoff beschickt. Die Analyse des Reaktoraustrags erfolgte wie vorstehend beschrieben. Der Katalysator wurde nach 59 Tagen vollständig erhalten ausgebaut. Die Ergebnisse sind in der nachstehenden Tabelle 1 zusammengefaßt.

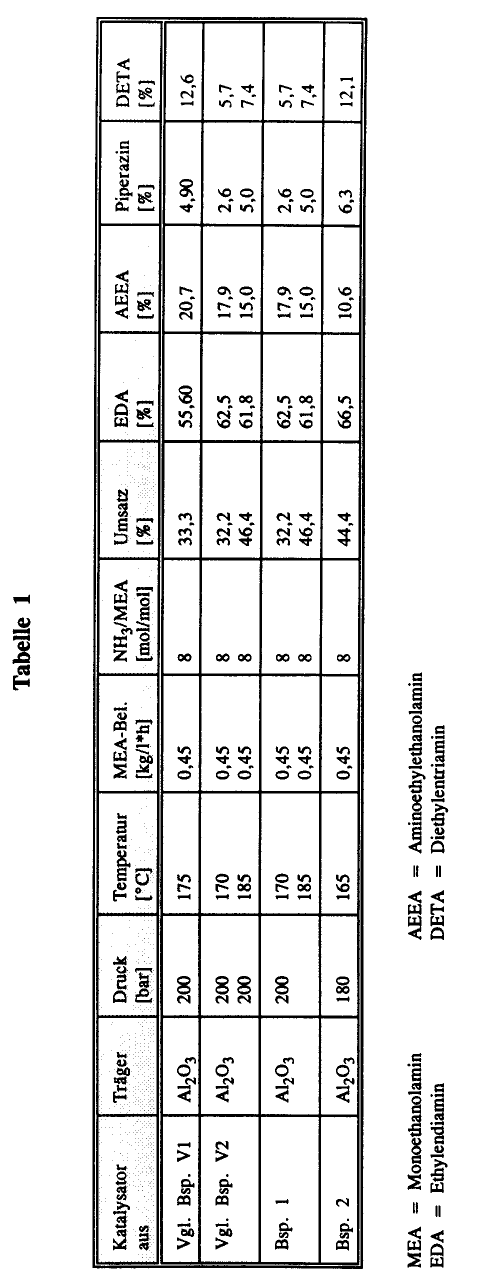

Aus den Ergebnissen der Tabelle 1 geht hervor, daß bei Verwendung der erfindungsgemäßen Katalysatoren die Selektivität in bezug auf Ethylendiamin deutlich höher ist als bei Verwendung der bekannten Katalysatoren. Zudem ist die Haltbarkeit der erfindungsgemäßen Katalysatoren wesentlich höher als die Haltbarkeit der Vergleichskatalysatoren.

## Patentansprüche

1. Katalysator, umfassend bezogen auf das Gesamtgewicht des Katalysators
mehr als 6 - 50 Gew.-% Kobalt, Nickel oder deren Gemisch,
0,001 - 25 Gew.-% Ruthenium,
0 - 10 Gew.-% Kupfer
und
0 - 5 Gew.-% Promotoren aus der Gruppe Eisen, Rhodium, Palladium, Platin, Iridium, Osmium, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder Gemischen davon
auf einem porösen Metalloxidträger, herstellbar durch
(a) Imprägnieren des Trägers mit den Metallen, Promotoren oder Verbindungen davon,
(b) Trocknen und Calcinieren des imprägnierten Trägers und
(c) Reduzieren des calcinierten Trägers im Wasserstoffstrom,
wobei der Träger nicht mit Halogenverbindungen imprägniert wird.

2. Katalysator nach Anspruch 1, umfassend mehr als 3 - 25 Gew.-% Kobalt und mehr als 3 - 25 Gew.-% Nickel.

3. Katalysator nach Anspruch 1 oder 2, wobei der poröse Metalloxidträger ausgewählt ist aus Aluminiumoxid, Siliciumdioxid, Alumosilicaten, Titandioxid, Zirkoniumdioxid, Magnesiumoxid oder Gemischen davon.

4. Katalysator nach einem der Ansprüche 1 bis 3, der im wesentlichen halogenfrei ist.

5. Verfahren zur Herstellung eines Katalysators nach einem der Ansprüche 1 bis 4 durch
(a) Imprägnieren des Trägers mit den Metallen, Promotoren oder Verbindungen davon,
(b) Trocknen und Calcinieren des imprägnierten Trägers und
(c) Reduzieren des calcinierten Trägers im Wasserstoffstrom.

6. Verwendung des Katalysators nach einem der Ansprüche 1 bis 4 in Hydrierungsreaktionen, Dehydrierungsreaktionen oder Hydrierungs-/Dehydrierungsreaktionen.

7. Verwendung nach Anspruch 6, wobei die Hydrierungs-/Dehydrierungsreaktion eine Aminierung von Alkylenoxiden, Alkoholen, Aldehyden oder Ketonen mit Ammoniak, primären oder sekundären Aminen ist.

8. Verfahren zur Herstellung von Aminierungsprodukten durch Umsetzung von Alkylenoxiden, Alkoholen, Aldehyden oder Ketonen mit Ammoniak, primären oder sekundären Aminen in Gegenwart von freiem Wasserstoff und in Gegenwart eines Katalysators nach einem der Ansprüche 1 bis 4.

9. Verfahren nach Anspruch 8, wobei Ethylenglykole oder Ethanolamine mit oder in Gegenwart von Ammoniak, Ethanolaminen, Ethylendiaminen oder Diethylentriaminen aminiert werden.

10. Verfahren nach Anspruch 8 oder 9, das kontinuierlich ausgeführt wird.
